# EUROPEAN PATENT SPECIFICATION

(11) **EP 3 123 874 B1**
(45) Date of publication and mention of the grant of the patent: **02.05.2018**
(21) Application number: 15382389.3
(22) Date of filing: 28.07.2015
(51) Int. Cl.: A23L 33/105, A61K 38/47, A61J 3/07, A61K 9/48

(54) **FORMULATIONS COMPRISING GLUCOSINOLATES AND MYROSINASE**
ZUSAMMENSETZUNGEN ENTHALTEND GLUCOSINOLATES UND MYROSINASE
COMPOSITIONS COMPRENANT DES GLUCOSINOLATES ET DE LA MYROSINASE

(43) Date of publication of application: 01.02.2017
(73) Proprietor: Fundación Tecnalia Research & Innovation, 20009 San Sebastian (Guipuzcoa) (ES)
(72) Inventor: SÁENZ GÓMEZ, Jessica, 01510 Miñano-Araba (ES); OLIVER GARCÍA, Laura, 01510 Miñano-Araba (ES); VILLARÁN VELASCO, María Carmen, 01510 Miñano-Araba (ES)
(74) Representative: Balder IP Law, S.L.

(56) References cited:
- EP-A1- 2 213 280
- WO-A1-2012/074412
- US-A1- 2004 191 366
- US-A1- 2008 226 772
- US-A1- 2008 311 192
- US-A1- 2013 323 225
- US-A1- 2015 118 306

## Description

### FIELD OF THE INVENTION

The present invention falls within the field of functional nutritional foodstuffs and supplements. It relates to a formulation which comprises microcapsules which comprise a core comprising an extract rich in glucosinolates obtained from crucifers and a biopolymer, and an enteric coating comprising myrosinase and a biopolymer. The invention also relates to a procedure for obtaining the same and to the use of the formulation as such, or in dietetic foodstuffs and supplements for humans or animals due to its content of glucosinolates, precursors of sulforaphane with immunostimulant activity.

### BACKGROUND OF THE INVENTION

Glucosinolates are a group of glucosides stored in the interior of the cellular vacuoles of all the vegetables of dicotyledonous plants, and are especially abundant in the Brassicaceae family (crucifers), examples of these vegetables are broccoli, Brussel sprouts, cauliflower, kale, turnips, radishes, watercress, etc., broccoli and Brussel sprouts standing out due to their content of glucosinolates. The content of glucosinolates of the plants depends on the species and on the environmental conditions and agricultural activities as well as on the process of transformation and preparation following the harvesting thereof.

Glucosinolates do not exhibit biological activity, however, there exists a reaction by way of which the myrosinase enzyme (thioglucoside glucohydrolase), which is also found in vegetable cells, is capable of catalyzing these components towards a variety of hydrolyzed products, including isothiocyanates and indoles.

Amongst these isothiocyanates, sulforaphane stands out with known immunostimulant activity which can play a protecting and preventing role against diseases with a high incidence such as cardiovascular diseases, lung and bladder cancer, tumors and gastrointestinal diseases or diabetes. Sulforaphane helps to increase the activity of the glutathione S-transferase enzyme which intervenes in the metabolism of glutathione, an important antioxidant of the human body.

Unfortunately, sulforaphane cannot be supplied directly in the diet with the intention of obtaining an immunostimulant effect since it is an unstable compound which makes this option unviable.

Both the glucosinolates, specifically and mainly glucoraphanin, and the myrosinase are segregated by the plant itself but cannot react in a spontaneous manner with each other in the interior of the plant since they are physically separated from each other. The reaction commences when the plant is chewed or cut since this action produces cellular damage which causes the two compounds to enter into contact. In addition, when the plants are cooked, the myrosinase is inactive (at least partially) and consequently, the production of sulforaphane reduces or even disappears.

Although the intestinal flora is capable of converting the glucosinolates, specifically the ingested glucoraphanin into sulforaphane, this process is not efficient in the absence of active myrosinase. Since the ingestion of glucosinolates is usually produced following the processing and/or cooking of the plant, the presence of active myrosinase is very low and the efficiency in the conversion process of ingested glucoraphanin into sulforaphane is also very low. Therefore, it is currently practically impossible to be able to ingest the recommended quantity of these compounds by way of the diet.

Recently, in the prior art, foodstuff developments have been disclosed which have attempted to solve this problem with the direct addition of glucosinolates and the enzyme. In this context, the application WO2012/074412 A1 describes an oral composition which consists of an encapsulated composition of plant powders or extract and of an enzyme. This alternative, however, does not detail an effective separation of the extract and the myrosinase, being able to react prior to arriving at the large intestine where it is assimilated, therefore the stability and functionality of the compounds is not guaranteed.

With the aim of achieving greater bioactivity of the glucosinolates, other solutions have been proposed to protect them up to their place of assimilation. These solutions range from direct extrusions of foodstuffs with the extracts included as is described in US 2008/0311192 A1 to direct encapsulation by spray dryer. Other solutions consist of formulates in which the extracts and the myrosinase are encapsulated separately, generally by means of a spray dryer (WO 2012/116018 A1, WO2013/179056 A1) or even combined by means of an encapsulation process of the extract and subsequent coating steps of one or various materials to achieve the appropriate inclusion of myrosinase in the product and avoid contact
thereof with the glucosinolates (EP 2213280 A1).

US 2013/323225 A1, US 2015/118306 A1 and US 2008/226772 A1 also disclose various methods of encapsulation. All these solutions require the use of two or more consecutive processes of encapsulating and coating to achieve a product with which a human or animal can be provided with sulforaphane, which complicates and increases the cost of the process to obtain the same.

In view of the foregoing, there exists the need in the prior art to provide a new simple and economic procedure which allows the obtaining of an alternative, stable formulation which allows the sulforaphane to be supplied effectively to a human or animal.

### DESCRIPTION OF THE DRAWINGS

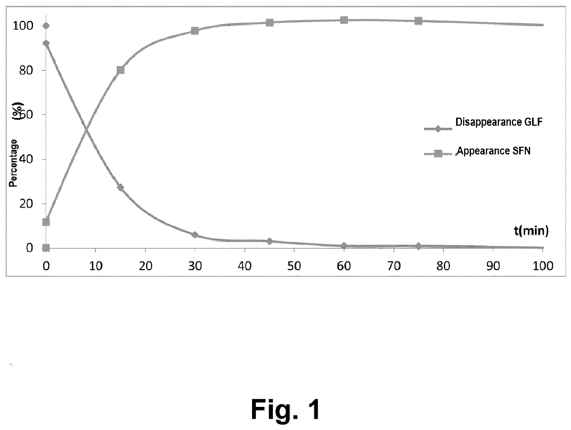

**Figure 1****:** the results of the UPLC-MS of Example 5 are shown.

### DESCRIPTION OF THE INVENTION

In a first aspect of the invention, it relates to a formulation according to the appeded claims. The core comprises a biopolymer, preferably a polysaccharide and more preferably alginate. The biopolymer is preferably of a foodstuff grade.

The extract rich in glucosinolates is obtained from plants of the Brassicaceae family (crucifers). Some examples of plants of this family are broccoli, Brussel sprouts, cabbage, cauliflower, kale, turnips, radishes, watercress, etc. In a preferred embodiment, the plant, from which the extract rich in glucosinolates is obtained, is broccoli.

The extract can be obtained from any part of the plant which contains glucosinolates, for example, the stalks or the florets. In a preferred embodiment, the stalks and/or the remains of florets are used, unprocessed by the foodstuffs industry which constitutes a by-product of this industry. In a more preferred embodiment, the stalks and/or the remains of broccoli florets
are used. This embodiment provides an additional advantage since it allows a by-product to be utilized, the accumulation of which constitutes a problem for the industry, revaluing it and generating a new source of additional income. Furthermore, the utilization thereof is aligned with the politics of efficient utilization of natural resources.

The term, "extract rich in glucosinolates" in the present invention should be understood in the sense that it contains a greater quantity of glucosinolates than that which the same weight of the natural product, from which it is obtained, has. The degree of enrichment of the extract varies depending on factors such as, for example the process itself of obtaining the extract, on the plant from which it is obtained, on the part of the plant, on whether it is concentrated, etc. There is no special limitation with respect to the degree of enrichment of the extract which can be used.

The extract rich in glucosinolates which is used in the present invention can be that which comes directly from an extraction process of the plant and can be the extract resulting from a subsequent clarifying, bleaching and/or concentrating, as is presented further below.

The coating comprises the myrosinase enzyme and at least one biopolymer. The coating can comprise more than one biopolymer, for example two, three or more. The coating should be enteric. In one particular embodiment the coating comprises a mixture of chitosan-alginate. In another particular embodiment it comprises pectin. In another particular embodiment it comprises a mixture of alginate and pectin.

The myrosinase can be acquired in commercial form (for example from Sigma Aldrich T4528)

The fact that the coating of the microcapsules is enteric allows the microcapsules to be able to travel through the gastrointestinal tract and arrive intact at the large intestine, where the assimilation of the sulforaphane takes place.

The relation by weight between the extract rich in glucosinolates and myrosinase in the formulation can vary between large margins. However, in one particular embodiment, the relation is such that the quantity of enzyme is in a relation of between 5-50 %, preferably between 10-45 %, more preferably between 20-40 % by weight with respect to the quantity of glucoraphanin present in the extract used in the core.

The relation by weight between the biopolymer of the core and the extract can vary in a wide range. Typically the relation is between 0.5 %-5 % of biopolymer and 95 %-99.5 % of extract, preferably 1 %-3 % of biopolymer and 97 %-99 % of extract.

The minimum number of layers of the microcapsules of the invention is two, one interior or core and one exterior or coating. However, the microcapsules can comprise one or more additional layers identical to or different from each other. According to one particular embodiment, the microcapsules have a third layer which covers the coating. In a more particular embodiment, this third layer can be applied to increase the stability of the microcapsules against temperature. This layer comprises, in one particular embodiment, a wax.

The presence of glucosinolates and myrosinase in separate layers avoids the interaction and therefore the reaction between these taking place, producing a stable formulation. The term, "stable", referring to a formulation, refers to the fact that the integrity of these precursors is guaranteed for the formulation of a bioavailable and functional sulforaphane, i.e. that the glucosinolates and the myrosinase remain intact until the formulation arrives to the large intestine, where the microcapsules break and where the assimilation of the sulforaphane is produced.

In addition however, it has been proven that the formulation is also stable against the temperature in a wide range. Thus, it has been observed that the formulation which comprises microcapsules which have only the core and the coating, it is advantageously stable at temperatures greater than 4 °C, for example at room temperature (typically between 15 and 30 °C).

The form of the formulation can be diverse according to the degree of moisture: in a wet state, recently obtained, it has the appearance of uniform spheres of gelatin and in the dry state it has the appearance of a powder.

The formulation can also comprise auxiliary compounds useful for the formulation which modify some of the properties thereof such as the storage stability, the visual perception, the physical stability, nutritional value, etc. The auxiliary compounds are also preferably selected from those compounds which can be used for the application thereof in foodstuffs and food supplements. The quantity of auxiliary compounds can vary as a function of each particular case between wide margins. Illustrative, but non-limiting examples of auxiliary compounds are anti-oxidants, colorants, flavorings, vitamins and mixtures of the same.

### Obtaining the extract:

The extract rich in glucosinolates can be prepared previously by any conventional procedure known to an expert, such as by extraction with a suitable solvent or by extraction with supercritical fluids.

Any part of the crucifer plant is useful such as for example florets, stalks, etc., although in one particular embodiment, it departs from a by-product of the plant, such as for example stalks and remains of florets. The use of the stalks and remains of broccoli florets constitutes an additional advantage in the procedure of the invention since these constitute remains or by-products of industrial processing into processed products (canned products, frozen products, etc.), and have an elevated content of glucosinolates. Therefore, the utilization of these by-products, suitably treated for avoiding the deterioration of their active substances, as primary material for obtaining the glucosinolate extract is an advantageous option which revalues these by-products and avoids the disposal thereof.

In one particular embodiment, the extract is obtained by solid/liquid extraction, where the solvent used is alcohol or a mixture of alcohol and water. In one preferred embodiment, the alcohol is ethanol. In one particular embodiment, the relation by volume alcohol:water is between 100:0 and 70:30, for example 80:20.

The extracts resulting from an extraction procedure can then be filtered to remove solid remains in suspension. At the same time, an obtained extract can optionally be clarified or bleached or clarified and bleached to remove the coloration thereof and/or to reduce impurities such as for example proteins.

The clarification of the extract is carried out by treatment of the same in acid medium. Specifically by means of the addition of an acid, for example hydrochloric acid, citric acid, ascorbic acid or acetic acid to reach an acidic pH of typically between 2 and 3. Then the resulting extract is left to precipitate, is centrifuged and the pH is returned to the original value thereof. The bleaching is carried out for example by making the solution of the extract pass through active carbon with the aim of removing the color of the solution and obtaining a transparent solution.

The bleaching and clarification can be carried out successively or in an individual manner.

### Procedure for obtaining the formulation:

The formulation of the invention is obtained by means of the application of the vibration technique with an encapsulation system. This technique is based on the principle that a stream of liquid which flows with a laminar flow through the interior of a nozzle is divided into droplets of equal size through the effect of a vibration. These microdrops fall into a gelification solution or bath appropriately formulated, producing the gelification thereof and consequent formation of the microcapsules.

In the case of the present invention, the encapsulation system used comprises a concentric dual nozzle system, such that at all times, two different solutions are made to circulate through the interior thereof, a first solution which produces the core and which is made to circulate through the interior nozzle and a second solution which produces the exterior layer and which is made to circulate simultaneously through the exterior nozzle. When the microdrops formed fall into the interior of a gelification solution, they produce the dual layer microcapsules of the core/coating type of the invention.

The procedure of the invention therefore comprises using an encapsulation system which comprises a concentric dual nozzle system and the steps of:
a) Preparing a first solution of at least one biopolymer and extract rich in glucosinolates;
b) Preparing a second solution of at least one biopolymer and myrosinase;
c) Making the first solution flow through the interior nozzle and the second solution through the exterior nozzle in a simultaneous manner and applying vibration to the stream of liquid which flows with a laminar flow; and
d) Letting the microdrops formed fall into a gelification bath and when appropriate, submerging the gelified microdrops in an additional solution which contains an enteric biopolymer.

In step a) for preparing a solution of at least one biopolymer and extract rich in glucosinolate, the biopolymer and extract proportion is as described previously. In one preferred embodiment, the biopolymer is an alginate and more preferably the relation by weight of alginate:extract is a relation of between 0.5 %-5 % of alginate and 99.5-95 % of extract, preferably between 1-3 % of alginate and 99-97 % of extract.

In step b) a second solution of at least one biopolymer and myrosinase is prepared. In one particular embodiment, said biopolymer is alginate. In another particular embodiment, the biopolymer is pectin.

For step c) any conventional encapsulation system can be used provided with a concentric dual nozzle system. The first solution prepared in a) is made to pass through the interior nozzle and the second solution prepared in b) through the exterior nozzle applying vibration such that microdrops are generated.

The size of the concentric nozzles can be varied and as a function of the same and of other parameters of the process, such as the frequency of vibration, the velocity of the fluid and the air pressure, the size of the microcapsules which are obtained can also be varied. Typically, the size of the nozzle is 200 µm the interior and 300 µm the exterior one. The first solution is loaded into the syringe of the system and is pumped through the interior nozzle controlling the velocity of the pump. Typical velocities of the pump are between 3 and 10 ml/min. The second solution is made to pass through the exterior nozzle controlling the flow by means of air pressure. The air pressure is generally between 0.05 and 0.2 MPa (0.5 and 2 bar).

The frequency of vibration which is applied to the concentric dual nozzle system can be varied, although generally it is between 1000 and 7000 Hz.

In the following step d), the microdrops are thus formed in a gelification bath which comprises calcium chloride. The gelification bath is maintained preferably with agitation to allow the suspension of the microcapsules which are formed therein.

The gelification bath can be easily prepared by a person skilled in the art as a function of what the second solution is and taking into account the enteric character which the microcapsules must have.

In this sense, if the biopolymer which is used for the second solution is not enteric (alginate, for example), it is necessary to incorporate an enteric biopolymer in the coating. As an enteric biopolymer, a biopolymer which then does not interact with calcium chloride can be incorporated, for example chitosan or a biopolymer which interacts with CICa2 such as, for example pectin. Thus, according to one particular embodiment, when chitosan is incorporated, the gelification bath can incorporate CICa2 and chitosan and it is not necessary to use any additional solution in step d). According to another particular embodiment, when a biopolymer is incorporated in the coating which interacts with CICa2 such as pectin, step d) comprises gelifying the microdroplets in a gelification bath which contains CICa2 and it is subsequently necessary to submerge them in a solution which contains said biopolymer (for example pectin).

Therefore, in one particular embodiment of the procedure, the second solution comprises alginate and myrosinase and the gelification bath comprises a solution with calcium chloride, chitosan and a weak acid for regulating the pH, such as for example acetic acid. A weak acid in the present invention refers to an acid which is not completely dissociated in an aqueous solution. In another particular embodiment, the second solution comprises pectin and the gelification bath comprises a solution with calcium chloride, preferably in a concentration equal to or greater than 9 % by weight.

In another particular embodiment, the second solution comprises alginate as biopolymer, the gelification bath comprises a solution with calcium chloride. In this case, the microcapsules are subsequently treated in an additional solution which comprises pectin for providing them with an enteric character.

The result of the procedure is the obtaining of a formulation according to the invention which comprises uniform and spherical microcapsules. Initially, the formulation is obtained wet.

The size of the wet microcapsules was determined and is typically between 300 and 1000 microns in diameter, although it is not limited to this range. The distribution of sizes is reduced and uniform in one particular embodiment.

In one particular embodiment, the wet size is between 600-900 microns. The size is not considered an essential characteristic of the microcapsules of the invention.

The procedure of the invention also comprises optionally an additional drying step. Drying reduces the activity of the water and increases the useful life of the microcapsules. The latter, following drying, have a powder form. Drying can be carried out in any conventional manner, such as fluid bed, lyophilization, drying by air, vacuum, etc. In one particular embodiment, fluid bed is used and is carried out at a temperature equal to or less than 30 °C, during a variable time, typically between 10 and 20 minutes.

In a preferred embodiment, the materials used for obtaining the formulation of the invention are of a foodstuff grade.

The formulation can be used in foodstuffs, both for humans and for animals and as a dietetic supplement.

Thus, in an additional aspect, the invention relates to the use of the formulation of the invention in a foodstuff.

In a further additional aspect, the invention relates to the use of the formulation of the invention in a dietetic supplement.

In the context of the invention, a foodstuff refers to liquid, semi-solid and solid food products, as well as in the form of paste or gel and similar. The food products comprises foodstuffs for human beings as well as for animals and constitute an additional aspect of the invention.

A dietetic supplement, also known as food supplement or nutritional supplement, is preparation intended to supply nutrients such as vitamins, minerals, fatty acids, amino acids etc., which are lacking or are not consumed in a sufficient quantity in the diet of a person or animal. In an additional aspect, the invention relates to a dietetic supplement which comprises the formulation of the invention.

The food product can be in a form ready to consume, which means a form which is suitable to eat without further processing. However, it is also possible that the food product is in a form which requires further processing to produce the product to be ingested, for example heating, dissolving, etc.

The food products intended for humans can be any food product. Illustrative examples are instant beverages, effervescent tablets, bars (cereals, chocolate), milk products, prepared foods or dehydrated foods such as packet soups, bars, snacks etc.

The food products for animals (feed products) can be in any commonly used form in this type of food.

### EXAMPLES

An encapsulation system was used: Inotech IE50-R from the company Inotech Encap Bio Systems Inc.

### EXAMPLE 1. Preparation of dual layer microcapsules (core/coating) of broccoli extract rich in glucosinolates with alginate in the core and myrosinase and chitosan alginate in the coating.

### Step 1. Stabilization and pre-treatment of the broccoli by-product

Blanching the broccoli stalks for 5-10 minutes at 80-95 °C, cooling with vaporized water and deep freezing for 5-15 min. Preserving at -20 °C until the processing thereof. Prior to carrying out the extraction, the frozen by-product is vacuum-dried in a number of ways, among them lyophilization and subsequently ground.

### Step 2: Obtaining the extract rich in glucosinolates

Obtaining the extract rich in glucosinolates by means of solid/liquid extraction using a solvent and the broccoli powder obtained in the previous step. The proportion of powder/liquid varies between 25-75 gr of powder per 2 liters of solvent. An extract with a concentration of glucosinolates of between 200 and 400 mg/l is thus obtained, the content of glucoraphanin being between 50-85 % by weight of the same.

The solvent used is an ethanol:water mixture in proportions 80:20. The extraction temperature was room temperature and the extraction time was 30 minutes.

### Step 3: Concentration of the extract

The extract obtained in step 2 is filtered to remove solids in suspension and the resulting solution is concentrated by means of vacuum distillation, at a temperature of between 20-40 °C and 3000-4000 Pa. The resulting extract has a concentration of between 1150 and 4000 mg/l of glucosinolates with a percentage of glucoraphanin of between 50 and 85 %.

### Step 4: Clarification of the extract

The clarification of the extract is optional and can be carried out by two procedures.

The first one was carried out by the treatment of the same in acid medium by means of the addition of hydrochloric acid, citric acid, ascorbic acid or acetic acid until a pH: 2-3 was reached. It was left to act between 20-40 mins until it precipitated, it was centrifuged at 4000 rpm for 15-30 minutes and it returned to the original pH of the sample with NaOH 0.1 M.

The second one was carried out with active carbon, making the extract pass through a Superclean ENVI-Carb SPE Tube commercial cartridge.

### Step 5: Microencapsulation of the extract

Developing microcapsules by way of the vibration technology previously described.

The microcapsules obtained have an alginate core and the extract rich in glucosinolates and a coating of alginate, chitosan and myrosinase.

For this, a solution of alginate and extract rich in glucosinolates from step 3 or 4 was used as the material for the core in a relation of alginate:extract of between 1:99 and 3:97 by weight. The materials used for the coating were a solution of alginate in a concentration of between of 1 and 3 % by weight and myrosinase present in a quantity such that the enzyme was found in a proportion of between 20 and 40 % by weight with respect to the quantity of glucoraphanin present in the extract used in the core.

The gelification bath incorporated between 1-3 % by weight of CaCl₂, between 0.2-1 % by weight of chitosan and acetic acid to achieve a solution of pH of between 3-4 which allowed the enteric microcapsules to be obtained.

The encapsulation parameters were:
- A concentric dual nozzle system: 200-300 µm in diameter the interior and exterior nozzle respectively
- Air pressure 0.05 and 0.1 MPa (0.5-1 bar)
- Pump velocity 200-300 (4-7.5 ml/min)
- Frequency of vibration 2000-5300 Hz

The dissolution mixture of alginate and extract rich in glucosinolates was made to pass through the interior nozzle and the dissolution of alginate with myrosinase was made to pass through the exterior nozzle.

In order to make the solution mixture of alginate and extract, alginate was added to the extract rich in glucosinolates and 50 ml was inserted into the syringe of the Intecho system and pumped through the interior nozzle, controlling the velocity of the pump.

Simultaneously, the dissolution of myrosinase and alginate was made to pass through the exterior part of the nozzle, controlling the flow by means of air pressure.

The drops formed by both solutions fell into a gelification bath in agitation, where the formation of the core/coating microcapsule took place.

### EXAMPLE 2. Preparation of dual layer microcapsules (core/coating) of broccoli extract rich in glucosinolates with alginate in the core and myrosinase and pectin alginate in the coating.

The steps 1, 2 and 3 were the same as in Example 1.

### Step 5: Microencapsulation of the extract

The microcapsule has a core of alginate and extract rich in glucosinolates and a coating of alginate, pectin and myrosinase.

A solution of alginate and extract from step 3 was used to form the core in a relation of alginate:extract of between 1:99 and 3:97 by weight. The materials used for the external layer were in a solution of alginate in a concentration of between 1 and 3 % by weight and myrosinase present in a quantity such that the enzyme was found in a proportion of between 20 and 40 % by weight with respect to the quantity of glucoraphanin present in the extract used in the core.

The gelification bath was a solution of between 1-3 % by weight of CaCl₂. Subsequently, the gelified microcapsules were submerged in a solution of between 0.1-1 % by weight of pectin to obtain enteric microcapsules.

The encapsulation parameters were the same as in Example 1.

### EXAMPLE 3. Preparation of dual layer microcapsules (core/coating) of broccoli_extract rich in glucosinolates with alginate in the core and myrosinase and pectin in the coating.

The steps 1, 2 and 3 were the same as in Example 1.

### Step 4: Microencapsulation of the extract

The microcapsule has a core of alginate and glucosinolates and a coating of pectin and myrosinase.

A solution of alginate and extract from step 3 was used for the core in a relation of alginate:extract of between 1:99 and 3:97 by weight. The materials used for the coating were in a solution of pectin in a concentration of between 3 and 6 % by weight and myrosinase present in a quantity such that the enzyme was found in a proportion of between 20 and 40 % by weight with respect to the quantity of glucoraphanin present in the extract used in the core.

The gelification bath consisted of a solution of CaCl₂ with a concentration of 10 %.

The encapsulation parameters were:
- A concentric dual nozzle system: 200-300 µm in diameter the interior and exterior nozzle respectively
- Air pressure 0.5-1 bar
- Pump velocity 200-300 (4-7.5 ml/min)
- Frequency of vibration 3000-7000 Hz

### EXAMPLE 4. Stability test of the formulation of the invention in the gastrointestinal tract

In order to achieve the correct functionality of the formulation it is necessary for the microcapsules to endure the acidic conditions of the stomach, remaining intact during their entire stay in the small intestine and the extract and the enzyme being released in the large intestine.

In order to verify this, the developed microcapsules were subjected to a gastric and intestinal simulation process.

Firstly, the microcapsules were dried using a Uni-Glatt model fluid bed from the company Glatt GmbH System. The maximum drying temperature was 30 °C for a maximum of 15 minutes.

For this test, it was carried out with the dried obtained microcapsules and the effect of the pH and of the bile salt on them was analyzed.

### -Effect of the pH:

An aqueous solution was prepared with 0.9 % by weight of NaCl, 3 g/l of pepsin and the pH was adjusted to 2 with 0.08 M HCI or 0.1 M NaOH. 25 milliliters of this solution was taken and 0.2 and 0.4 g of microcapsules obtained according to Example 1 was added.

They were maintained under agitation for two hours at 37 °C. An aliquot is then collected and the analysis of the compounds of interest (glucoraphanin and sulforaphane) is carried out by ultra pressure liquid chromatography in tandem with mass spectrometry (UPLC-MS: Acquity H-class Waters)

No presence of glucoraphanin or sulforaphane was detected, which means that the microcapsules had not broken and remained stable.

### -Effect of bile salt:

A solution of 3 g of bile was prepared in a litre of an intestinal solution 6.5 g/l NaCl; 0.835 g/l KCI, 0.22 g/l CaCl₂ and 1.386 g/l NaHCO₃) at pH 7.5 adjusted with HCI and NaOH.

25 millilitres of this solution was added to the solution from the analysis of the effect of the pH of the previous section and maintained in agitation for 2 hrs at 37 °C. A sample of the solution was taken for analyzing the compounds (glucoraphanin and sulforaphane) in the UPLC-MS. No presence of glucoraphanin or of sulforaphane was detected, which presumes that the microcapsules had not broken and remained stable.

The microcapsules were separated from the medium and were added in a solution of phosphate buffer, simulating the large intestine, where they remained in agitation at 37 °C. The subsequent analysis of the medium and specifically of the content of glucoraphanin and sulforaphane demonstrated that the capsules had broken. This reveals the enteric character of the same.

### EXAMPLE 5. Breaking dried microcapsules and analysis of the myrosinase extract reaction for producing sulforaphane.

Between 0.5 and 1 g of microcapsules obtained according to Example 1 were added to 25 ml of sodium citrate 0.1 M in order to achieve the total breaking of the microcapsules.

In a parallel manner, the compounds generated upon putting the extract rich in glucoraphanin and the myrosinase into contact at 37 °C were analyzed by UPLC-MS. It was observed how the glucoraphanin (GLF) was progressively reacting with the myrosinase and reducing the presence thereof and how it was forming and detecting the sulforaphane (SFN). (see Figure 1 where the percentage by weight of the compounds GLF and SFN are depicted against the time (minutes).

## Claims

1. A formulation which comprises microcapsules which comprise two layers: (i) one core or interior central part comprising an extract rich in glucosinolates and at least one biopolymer, being a polysaccharide and (ii) one enteric coating or exterior layer surrounding the core comprising myrosinase and at least one biopolymer.

2. Formulation according to claim 1, wherein the extract rich in glucosinolates is obtained from a crucifer plant.

3. Formulation according to claim 2, wherein the plant is broccoli.

4. Formulation according to claim 1 or 3, wherein the polysaccharide of the core is alginate.

5. Formulation according to any one of claims 1 to 4, wherein the biopolymer of the coating is selected from (i) chitosan alginate, (ii) pectin, and (iii) pectin alginate.

6. Formulation according to any one of claims 1 to 5, wherein the relation by weight between the extract rich in glucosinolates and myrosinase is such that the quantity of myrosinase is in a relation of between 5-50 % by weight with respect to the quantity of glucoraphanin present in the extract used in the internal layer.

7. Formulation according to claim 6, wherein the relation by weight between the extract rich in glucosinolates and myrosinase is such that the quantity of myrosinase is in a relation of between 10-45 %, more preferably between 20-40 % by weight with respect to the quantity of glucoraphanin present in the extract used in the core.

8. Formulation according to any one of claims 1 to 7, wherein the relation by weight between the biopolymer of the core and the extract is between 0.5 %-5 % of biopolymer and 95 %-99.5 % of extract.

9. Formulation according to any one of claims 1 to 8, which comprises a third layer which covers the coating with wax.

10. Procedure for obtaining the formulation according to any one of the preceding claims which comprises using an encapsulation system comprising a concentric dual nozzle system and the steps of:
a) Preparing a first solution of at least one biopolymer being a polysaccharide and extract rich in glucosinolates;
b) Preparing a second solution of at least one biopolymer and myrosinase;
c) Making the first solution flow through the interior nozzle and the second solution through the exterior nozzle in a simultaneous manner and applying vibration to the stream of liquid which flows with a laminar flow; and
d) Letting the microdrops formed fall into a gelification bath and when appropriate, submerging the gelified microdrops in an additional solution which contains an enteric biopolymer.

11. A procedure according to claim 10, wherein the second solution comprises alginate and myrosinase, and the gelification bath comprises calcium chloride, chitosan and weak acid.

12. A procedure according to claim 10, wherein the second solution comprises pectin and myrosinase, and the gelification bath comprises calcium chloride.

13. A procedure according to claim 10, wherein the second solution comprises alginate and myrosinase, the gelification bath comprises a solution of calcium chloride and the gelified microcapsules are submerged in an additional solution which comprises pectin.

14. Use of the formulation according to any one of the preceding claims 1 to 9, in a foodstuff for humans or animals, or a food supplement.

15. Human or animal foodstuff or dietetic supplement which comprises the formulation according to any one of claims 1 to 9.

## Patentansprüche

1. Eine Formulierung, die Mikrokapseln umfasst, die zwei Schichten umfassen: (i)einen Kern bzw. einen zentralen Innenteil, der einen an Glucosinolaten reichen Extrakt und zumindest ein Biopolymer umfasst, wobei es sich um ein Polysaccharid handelt, und (ii)eine magensaftresistente Beschichtung bzw. eine den Kern umgebende Außenschicht, die Myrosinase und zumindest ein Biopolymer umfasst.

2. Formulierung nach Anspruch 1, wobei der an Glucosinolaten reiche Extrakt von einer Kreuzblütlerpflanze erhalten wird.

3. Formulierung nach Anspruch 2, wobei die Pflanze Brokkoli ist.

4. Formulierung nach Anspruch 1 oder 3, wobei das Polysaccharid des Kerns Alginat ist.

5. Formulierung nach einem der Ansprüche 1 bis 4, wobei das Biopolymer der Beschichtung ausgewählt ist aus (i) Chitosanalginat, (ii) Pektin und (iii) Pektinalginat.

6. Formulierung nach einem der Ansprüche 1 bis 5, wobei das Gewichtsverhältnis zwischen dem an Glucosinolaten reichen Extrakt und Myrosinase so ist, dass die Menge an Myrosinase in einem Verhältnis zwischen 5-50 Gew .-% in Bezug auf die Menge an Glucoraphanin liegt, welches in dem in der inneren Schicht verwendeten Extrakt vorhanden ist.

7. Formulierung nach Anspruch 6, wobei das Gewichtsverhältnis zwischen dem an Glucosinolaten reichen Extrakt und der Myrosinase so ist, dass die Menge an Myrosinase in einem Verhältnis zwischen 10-45 Gew.-%, bevorzugter zwischen 20-40 Gewichts-%, bezogen auf die Menge an Glucoraphanin liegt, welches in dem im Kern verwendeten Extrakt vorhanden ist.

8. Formulierung nach einem der Ansprüche 1 bis 7, wobei das Gewichtsverhältnis zwischen dem Biopolymer des Kerns und dem Extrakt zwischen 0,5% -5% Biopolymer und 95% -99,5% Extrakt liegt.

9. Formulierung nach einem der Ansprüche 1 bis 8, die eine dritte Schicht umfasst, die die Beschichtung mit Wachs bedeckt.

10. Verfahren zum Erhalten der Formulierung nach einem der vorigen Ansprüche, umfassend die Verwendung eines Verkapselungssystems, das ein konzentrisches Doppeldüsensystem umfasst, sowie die folgenden Schritte:
a) Herstellen einer ersten Lösung aus zumindest einem Biopolymer, wobei es sich um Polysaccharid handelt, und einem Extrakt, der reich an Glucosinolaten ist;
b) Herstellen einer zweiten Lösung aus zumindest einem Biopolymer und Myrosinase;
c) Gleichzeitiges Strömenlassen der ersten Lösung durch die innere Düse und der zweiten Lösung durch die äußere Düse und Beaufschlagen des mit einer laminaren Strömung strömenden Flüssigkeitsstroms mit Vibration; und
d) Fallenlassen der gebildeten Mikrotropfen in ein Gelierungsbad und gegebenenfalls Eintauchen der gelierten Mikrotröpfchen in eine zusätzliche Lösung, die ein magensaftresistentes Biopolymer enthält.

11. Ein Verfahren nach Anspruch 10, wobei die zweite Lösung Alginat und Myrosinase umfasst und das Gelierungsbad Calciumchlorid, Chitosan und schwache Säure umfasst.

12. Ein Verfahren nach Anspruch 10, wobei die zweite Lösung Pektin und Myrosinase umfasst und das Gelierungsbad Calciumchlorid umfasst.

13. Ein Verfahren nach Anspruch 10, wobei die zweite Lösung Alginat und Myrosinase umfasst, das Gelierungsbad eine Lösung aus Calciumchlorid umfasst und die gelierten Mikrokapseln in eine zusätzliche Lösung eingetaucht werden, die Pektin umfasst.

14. Verwendung der Formulierung nach einem der vorigen Ansprüche 1 bis 9 in einem Nahrungsmittel für Menschen oder Tiere oder einem Nahrungsergänzungsmittel.

15. Menschliches oder tierisches Nahrungsmittel oder diätetisches Ergänzungsmittel, welches die Formulierung nach einem der Ansprüche 1 bis 9 umfasst.

## Revendications

1. Formulation comprenant des microcapsules qui comprennent deux couches : (i) un noyau ou partie centrale intérieure comprenant un extrait riche en glucosinolates et au moins un biopolymère, qui est un polysaccharide, et (ii) un revêtement gastro-résistant ou couche extérieure entourant le noyau et comprenant de la myrosinase et au moins un biopolymère.

2. Formulation selon la revendication 1, dans laquelle l'extrait riche en glucosinolates est obtenu à partir d'une plante crucifère.

3. Formulation selon la revendication 2, dans laquelle la plante est du brocoli.

4. Formulation selon la revendication 1 ou 3, dans laquelle le polysaccharide du noyau est de l'alginate.

5. Formulation selon l'une quelconque des revendications 1 à 4, dans laquelle le biopolymère du revêtement est choisi parmi (i) le chitosane alginate, (ii) la pectine et (iii) la pectine alginate.

6. Formulation selon l'une quelconque des revendications 1 à 5, dans laquelle le rapport pondéral entre l'extrait riche en glucosinolates et la myrosinase est tel que la quantité de myrosinase est comprise entre 5 et 50 % en poids par rapport à la quantité de glucoraphanine présente dans l'extrait utilisé dans la couche interne.

7. Formulation selon la revendication 6, dans laquelle le rapport pondéral entre l'extrait riche en glucosinolates et la myrosinase est tel que la quantité de myrosinase est comprise entre 10 et 45 %, de préférence entre 20 et 40 % en poids par rapport à la quantité de glucoraphanine présente dans l'extrait utilisé dans le noyau.

8. Formulation selon l'une quelconque des revendications 1 à 7, dans laquelle le rapport pondéral entre le biopolymère du noyau et l'extrait est compris entre 0,5 % et 5 % de biopolymère et 95 % à 99,5 % d'extrait.

9. Formulation selon l'une quelconque des revendications 1 à 8, laquelle comprend une troisième couche qui recouvre de cire le revêtement.

10. Procédé pour obtenir la formulation selon l'une quelconque des revendications précédentes qui comprend l'utilisation d'un système d'encapsulation comprenant un système concentrique à double buse et les étapes consistant :
a) à préparer une première solution d'au moins un biopolymère qui est un polysaccharide et d'un extrait riche en glucosinolates ;
b) à préparer une seconde solution d'au moins un biopolymère et de myrosinase ;
c) à faire s'écouler la première solution à travers la buse intérieure et la seconde solution à travers la buse extérieure de façon simultanée, et à appliquer une vibration au courant de liquide s'écoulant selon un écoulement laminaire ; et
d) à laisser tomber les microgouttes formées dans un bain de gélification et, le cas échéant, à immerger les microgouttes gélifiées dans une solution supplémentaire contenant un biopolymère gastro-résistant.

11. Procédé selon la revendication 10, dans lequel la seconde solution comprend de l'alginate et de la myrosinase, et le bain de gélification comprend du chlorure de calcium, du chitosane et un acide faible.

12. Procédé selon la revendication 10, dans lequel la seconde solution comprend de la pectine et de la myrosinase, et le bain de gélification comprend du chlorure de calcium.

13. Procédé selon la revendication 10, dans lequel la seconde solution comprend de l'alginate et de la myrosinase, le bain de gélification comprend une solution de chlorure de calcium, et les microcapsules gélifiées sont immergées dans une solution supplémentaire comprenant de la pectine.

14. Utilisation de la formulation selon l'une quelconque des revendications précédentes 1 à 9, dans un produit alimentaire destiné à l'homme ou à l'animal, ou dans un complément alimentaire.

15. Produit alimentaire humain ou animal ou complément diététique comprenant la formulation selon l'une quelconque des revendications 1 à 9.
